# EUROPEAN PATENT APPLICATION

(11) **EP 1 552 824 A1**
(43) Date of publication of application: **13.07.2005**
(21) Application number: 03756658.5
(22) Date of filing: 16.10.2003
(51) Int. Cl.: A61K 31/166, A61P 11/00, A61P 43/00

(54) **THERAPEUTIC AGENT FOR CHRONIC OBSTRUCTIVE PULMONARY DISEASE**

(30) Priority: 17.10.2002 JP 2002302564; 09.07.2003 JP 2003272600
(71) Applicant: ONO PHARMACEUTICAL CO., LTD., Osaka-shi, Osaka 541-8526 (JP)
(72) Inventor: YOSHIZAWA, Toshio, Ono Pharmaceutical Co.Ltd., Mishima-gun,Osaka 618-8585 (JP); UEGAKI, Akihiko, Ono Pharmaceutical Co.Ltd., Mishima-gun,Osaka 618-8585 (JP)
(74) Representative: Henkel, Feiler & Hänzel
(86) International application number: PCT/JP2003/013261
(87) International publication number: WO 2004/035038

(57) **Abstract**

A prevention and/or treatment agent for chronic obstructive pulmonary disease comprising a hydroxamic acid compound of formula (I) 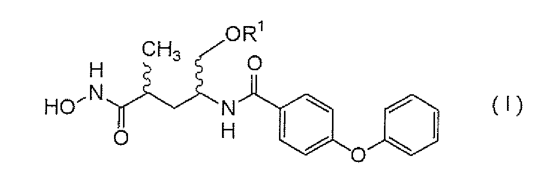 wherein R¹ is (i) hydrogen, (ii) C1-8 alkyl or (iii) C1-8 alkyl substituted by -OR², in which R² is (i) hydrogen, (ii) C1-8 alkyl, (iii) benzyl, or (iv) C1-8 alkyl substituted by C1-8 alkoxy; a salt thereof, a solvate thereof or a prodrug thereof as active ingredient.

## Description

### Technical Field

This invention relates to a prevention and/or treatment agent for chronic obstructive pulmonary disease. More particularly, this invention relates to a prevention and/or treatment agent for chronic obstructive pulmonary disease comprising a hydroxamic acid compound of formula (I) 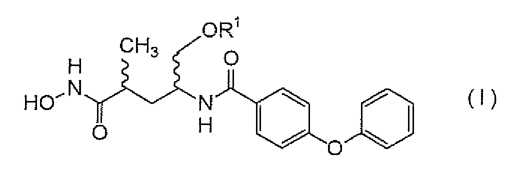 wherein all symbols are as hereinafter;
a salt thereof, a solvate thereof or a prodrug thereof as active ingredient.

### Background Art

Chronic obstructive pulmonary disease (COPD) is a generic name of a disease that causes breathing difficulties by the airway blockage of irreversible. It was caused that the inflammation in a bronchial tube, a minute bronchial tube and alveoli was occurred, and the flow of air to lungs was worsened chronic due to those inflammations and the elasticity decreases of lungs. Majority of the COPD patients exhibit a feature of pulmonary emphysema, chronic bronchitis and / or bronchial asthma, and it is difficult to distinguish them. However, presently, it is avoided to assume three above-mentioned diseases to be COPD, and it is assumed a typical disease to have to use a peculiar diagnosis name respectively. Anticholinergic drug, the β2 stimulation medicine and Teofirin, *etc.* are used for the treatment of COPD now, however, they are not satisfied enough because they have some proulem of a side effect.

On the other hand, in the specification of WO 99/19296, aminobutyric acid derivatives of formula (Z) 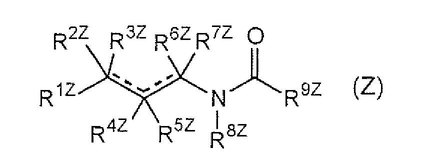 wherein R^{1Z} is -COOR^{10Z}, -CONHOR^{10Z}, *etc*.; R^{10Z} is hydrogen, C1-8 alkyl, *etc.;* R^{2Z}, R^{3Z}, R^{4Z}, R^{5Z}, R^{6Z}, and R^{7Z} each, in dependently, is (1) hydrogen, (2) C1-8 alkyl, (3) C2-8 alkenyl, (4) -OR^{11Z}, *etc*.; when R^{8Z} is (1) hydrogen, (2) C1-8 alkyl, then R^{9Z} is 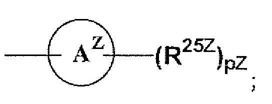 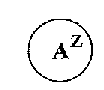 is a carbocyclic ring or a heterocyclic ring;
R^{25Z} is -E^{Z}-G^{Z} ; E^{Z} is (1) a single bond, (2) -CONR^{33Z}, (3) -NR^{33Z}CO-, (4) -CO-O-, *etc*.; G^{Z} is (1) hydrogen, (2) C1-8 alkyl, (3) Cyc4^{Z}, *etc*.;
having a matrix metalloproteinase (MMP) inhibitory activity, was reported. And the compound was disclosed to be useful for a treatment and/or prevention of rheumatoid disease, arthrosteitis, unusual bone resorption, osteoporosis, periodontitis, interstitial nephritis, arteriosclerosis, pulmonary emphysema, cirrhosis, cornea injury, metastasis of, invasion of or growth of tumor cells, autoimmune diseases (e.g. Crohn's disease, Sjogren's syndrome), diseases caused by vascular emigration or in filtration of leukocytes, arterialization, multiple sclerosis, aorta aneurysm, endometriosis.

However, there was not a description and a suggestion in the above specification that a compound of formula (Z) is useful for COPD.

Besides, an application of a compound having a MMP inhibitory activity to a treatment of COPD is not established as a generically treatment method.

### Disclosure of the Invention

Under the circumstances that a supply of a prevention and/or treatment agent for COPD is desired, energetic investigations have been carried out in order to accomplish the above purpose by the present inventors. They have found first that a hydroxamic acid compound of formula (I) or a salt thereof, a solvate thereof or a prodrug thereof is useful for COPD, and accomplished the present invention.

This invention relates to a prevention and/or treatment agent for COPD comprising a hydroxamic acid compound of formula (I) 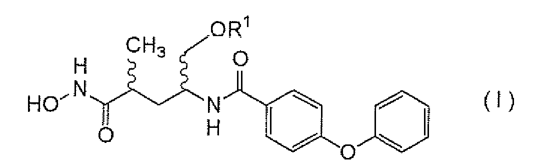 wherein R¹ is (i) hydrogen, (ii) C1-8 alkyl or (iii) C1-8 alkyl substituted by -OR², in which R² is (i) hydrogen, (ii) C1-8 alkyl, (iii) benzyl, or (iv) C1-8 alkyl substituted by C1-8 alkoxy; or
a salt thereof, a solvate thereof or a prodrug thereof as active ingredient.

In the present invention, C1-8 alkyl is straight-chain or branched-chain C1-8 alkyl selected from methyl, ethyl, propyl, isopropyl, butyl, isobutyl, sec-butyl, tert-butyl, pentyl, isopentyl, neopentyl, hexyl, isohexyl, heptyl and octyl.

In the present invention, C1-8 alkyl substituted by -OR² is straight-chain or branched-chain C1-8 alkyl selected from methyl, ethyl, propyl, isopropyl, butyl, isobutyl, sec-butyl, tert-butyl, pentyl, isopentyl, neopentyl, hexyl, isohexyl, heptyl and octyl, which substituted by one of -OR².

In the present invention, C1-8 alkyl substituted by C1-8 alkoxy is straight-chain or branched-chain C1-8 alkyl selected from methyl, ethyl, propyl, isopropyl, butyl, isobutyl, sec-butyl, tert-butyl, pentyl, isopentyl, neopentyl, hexyl, isohexyl, heptyl and octyl, which substituted by one of straight-chain or branched-chain C1-8 alkoxy selected from methoxy, ethoxy, propoxy, isopropoxy, butoxy, isobutoxy, sec-butoxy, tert-butoxy, pentyloxy, isopentyloxy, neopentyloxy, hexyloxy, isohexyloxy, heptyloxy and octyloxy.

In the present invention, 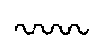 shows that the bond is an isomer resulting from the presence of an asymmetric carbon or a mixture thereof. Concretely, it shows the bond is in front of paper, that is, α-configuration 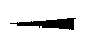 , the bond is on the other side of paper, that is, β-configuration 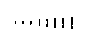 or a mixture thereof.

Unless otherwise specified, all isomers are included in the present invention. For example, alkyl and alkoxy include straight and branched isomers. Isomers resulting from the presence of asymmetric carbon(s) (R-configuration, S-configuration, α-configuration, β-configuration, enantiomers, diastereoisomers), optically active compounds having optical rotation (D, L, d, l-configuration), polar compounds obtained by chromatographic separations (highly polar compound, less polar compound), equilibrium compounds, the mixtures are existed by free ratio, racemic mixtures are included in the present invention.

In the present invention, all groups represented by R¹ are preferable. More preferable group is (i) hydrogen, (ii) C1-4 alkyl or (iii) C1-4 alkyl substituted by one of -OR², and preferable R² is (i) hydrogen, (ii) C1-4 alkyl, (iii) benzyl, or (iv) C1-4 alkyl substituted by one of C1-4 alkoxy. Especially preferable R¹ is (i) hydrogen or (ii) C1-4 alkyl substituted by one of -O-(C1-4 alky).

C1-4 alkyl is methyl, ethyl, propyl, isopropyl, butyl, isobutyl, sec-butyl or tert-butyl.

C1-4 alkoxy is methoxy, ethoxy, propoxy, isopropoxy, butoxy, isobutoxy, sec-butoxy or tert-butoxy.

Especially methyl and ethyl are preferable.

In the compound of formula (I) of the present invention, the compound of formula (Ia) 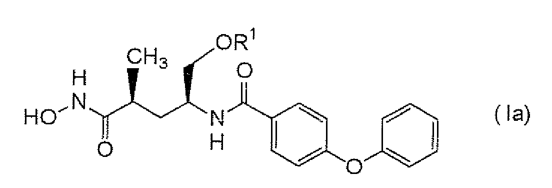 wherein all symbols are as hereinbefore;
a salt thereof, a solvate thereof or a prodrug thereof is preferable.

In the present invention, concretely compounds are
N-hydroxy-5-hydroxy-2(S)-methyl-4(S)-(4-phenoxybenzoyl)aminopentanamide,
N-hydroxy-5-methoxymethyloxy-2(S)-methyl-4(S)-(4-phenoxybenzoyl)aminopentanamide,
N-hydroxy-5-ethoxymethyloxy-2(S)-methyl-4(S)-(4-phenoxybenzoyl)aminopentanamide,
N-hydroxy-5-ethoxymethyloxy-2(R)-methyl-4(R)-[N-(4-phenoxyphenylcarbonyl)amino]pentanamide,
N-hydroxy-5-ethoxymethyloxy-2(R)-methyl-4(S)-[N-(4-phenoxyphenylcarbonyl)amino]pentanamide,
N-hydroxy-5-benzyloxymethyloxy-2(S)-methyl-4(S)-(4-phenoxybenzoyl)aminopentanamide, and
N-hydroxy-5-(2-metoxyethoxy)methyloxy-2(S)-methyl-4(S)-(4-phenoxybenzoyl)aminopentanamide,
a salt thereof, a solvate thereof or a prodrug thereof.

Especially preferable compounds are
N-hydroxy-5-hydroxy-2(S)-methyl-4(S)-(4-phenoxybenzoyl)aminopentanamide or
N-hydroxy-5-ethoxymethyloxy-2(S)-methyl-4(S)-(4-phenoxybenzoyl)aminopentanamide,
a salt thereof, a solvate thereof or a prodrug thereof.

The compound of the present invention may be used a following salt. Non-toxic and water-soluble salts are preferable. Suitable salts, for example, include: salts of alkali metals (e.g. potassium, sodium), salts of alkaline earth metals (e.g. calcium, magnesium), ammonium salts, salts of pharmaceutically acceptable organic amines (e.g. tetramethylammonium, triethylamine, methylamine, dimethylamine, cyclopentylamine, benzylamine, phenethylamine, piperidine, monoethanolamine, diethanolamine, tris(hydroxymethyl)amine, lysine, arginine, N-methyl-D-glucamine).

The solvates are preferably non-toxic and water-soluble. The appropriate solvates include, for example, solvates such as water, alcohol solvents (ethanol, etc.), and the like. Besides, the solvates of alkali (alkaline earth) metal salts, ammonium salts, organic amine salts, and acid addition salts of the compound are included.

In the present invention, the prodrug that it is improved an extent of bioavailability and a perviousness of biomembrane is preferable. Because the present compound is a hydroxamic acid compound, the prodrug means a compound which is converted to a hydroxamic acid compound by cleavage in the living body.

In the case of a compound wherein R¹ is hydroxyl or a group including hydroxyl, the prodrug means a compound having a group which is converted to hydroxyl by cleavage in the living body.

As a compound having a group which is converted to hydroxamic acid or hydroxyl by cleavage in the living body, for example, it is include a compound having acylated, alkylated, phosphorylated or borated hydroxyl. Concretely, a compound having acetylated, palmitoyl, propanoyl, pivaloyl, succinyl, fumaryl, alanyl or dimethylaminomethylcarbonyl in stead of hydroxyl in hydroxamic acid or other hydroxyl groups, are given.

These compounds are prepared by per se known method. In addition, the prodrug of the present compound may solvate or non-solvate.

The compound of formula (I) may be prepared, for example, by a method in a specification of WO 99/19296.

### Toxicity:

The toxicity of the compounds of the present invention is very low and therefore, the compounds may be considered safe for pharmaceutical use. For example, a minimum of lethal dose of N-hydroxy-5-hydroxy-2(S)-methyl-4(S)-(4-phenoxybenzoyl)aminopentanamide and N-hydroxy-5-ethoxymethyloxy-2(S)-methyl-4(S)-(4-phenoxybenzoyl)aminopentanamide by single oral administration to rat was 2000 mg/kg.

### Application for Pharmaceuticals:

The hydroxamic acid compound, a salt thereof, a solvate thereof or a prodrug thereof in the present invention, is useful for a prevention and/or treatment of COPD, in animals including human beings, especially human beings.

The compound of formula (I), a salt thereof, a solvate thereof or a prodrug thereof may be administered in combination with other medicaments for the purpose of
1) complement and/or enhancement of preventing and/or treating effect,
2) improvement of dynamics and absorption of the compound, and lowering of dose, and/or
3) alleviation of side effect of the compound.

The compound of formula (I) may be administered in combination with other medicaments as a composition in one drug product comprising these components, or may be administered separately. When they are administered independently, they may be administered simultaneously or with time lag. Administering with time lag includes the method of administering the compound of formula (I) before other medicaments and vice versa, and they may be administered in the same route or not.

The above combination takes effects on whichever disease treating and/or preventing effect of the compound of formula (I) is complemented and/or enhanced.

As other medicaments to complement and/or to enhance the preventing and/or treating effect of the compound of formula (I) for COPD, for example, anticholinergic agent, β₂ stimulator, theophylline products, leukotriene receptor antagonist, thromboxane syrzthetase inhibitor, thromboxane A₂ receptor antagonist, mediator release inhibitor, antihistamic agent, xanthine derivative, cytokine inhibitor, prostaglandins, forskolin, phosphodiesterase inhibitor, elastase inhibitor, aerosol steroid, expectorant, antibacterial agent.

As anticholinergic agent, for example, Ipratropium bromide, Oxitropium bromide, Flutropium bromide, Cimetropium bromide, Temiverine, Tiotropium bromide, Revatropate (UK-112166) are given.

As β₂ stimulator, for example, Fenoterol hydrobromide, Salbutamol sulfate, Terbutaline sulfate, Formoterol fumarate, Salmeterol xinafonate, Isoproterenol sulfate, Orciprenaline sulfate, clorprenaline sulfate, Epinephrine, Trimetoquinol hydrochloride, Hexoprenaline sulfate, Procaterol hydrochloride, Tulobuterol hydrochloride, Tulobuterol, Pirbuterol hydrochloride, Clenbuterol hydrochloride, Mabuterol hydrochloride, Ritodrine hydrochloride, Bambuterol, Dopexamine hydrochloride, Meluadrine tartrate, AR-C68397, levosalbutamol, R,R-Formoterol, KUR-1246, KUL-7211, AR-C89855, S-1319 are given.

As theophylline products, for example, Theophylline, Aminophylline are given.

As leukotriene receptor antagonist, for example, Pranlukast hydrate, Montelukast, Zafirlukast, Seratrodast, MCC-847, KCA-757, CS-615, YM-158, L-740515, CP-195494, LM-1484, RS-635, A-93178, S-36496, BIIL-284, ONO-4057 are given.

As thromboxane A₂ receptor antagonist, for example, Seratrodast, Ramatroban, Domitroban calcium hydrate, KT-2-962 are given.

As elastase inhibitor, for example, ONO-5046, ONO-6818, MR-889, PBI-1101, EPI-HNE-4, R-665, ZD-0892, ZD-8321, GW-311616, AE-3763 are given.

As aerosol steroid, for example, Beclomethasone dipropionate, Fluticasone propionate, Budesonide, Flunisolide, Triamcinolone, ST-126P, Ciclesonide, Dexamethasone palmitate, Mometasone furoate, Prasterone sulfonate, Deflazacort, Methylprednisolone suleptanate, Methylprednisolone sodium succinate are given.

As expectorant, for example, Foeniculated ammonia spirit, Sodium acid carbonate, Bromhexine hydrochloride, Carbocysteine, Axnhroxol hydrochloride, Slow release ambroxol hydrochloride, Methylcysteine hydrochloride, Acetylcysteine, L-Cysteine ethyl ester hydrochloride, tyloxapol are given.

Weight ratio of the compound of formula (I) and other medicaments is not limited.

A combination of any two or more of other medicaments selected from same groups having same mechanism and/or different groups having different mechanism, may be administered.

In other medicaments to complement and/or to enhance the preventing and/or treating effect of the compound of formula (I), medicaments that not only exist now but also may be found in the future on the basis of above mechanisms are included.

For the purpose described above, the compound of formula (I) of the present invention or a concomitant drug combined the compound of formula (I) with other medicaments may be normally administered systemically or topically, usually by oral or parenteral administration.

The doses to be administered are determined depending upon, for example, age, body weight, symptom, the desired therapeutic effect, the route of administration, and the duration of the treatment, *etc*. In the human adult, the doses per person at a time are generally from 1 mg to 1000 mg, by oral administration, up to several times per day, and from 0.1 mg to 100 mg, by parenteral administration (preferably intravenous administration), up to several times per day, or continuous administration between 1 and 24 hours per day into vein.

As mentioned above, the doses to be used depend upon various conditions. Therefore, there are cases wherein doses lower than or greater than the ranges specified above may be used.

A preferable amount by oral administration is an amount selected from (i) 12.5 mg/ one solid composition, (ii) 25 mg/ one solid composition, (iii) 50 mg/ one solid composition and (iv) 100 mg/ one solid composition, or an amount by 1-3 times administration per day and 1-4 tablets per one administration, which are selected and combined from the above solid compositions, that is 1-12 tablets per day, for the underlying a condition of target disease. More preferable amount in the human adult is 12.5 mg to 600 mg per day.

The compound of formula (I) or concomitant drug combined the compound of formula (I) with other medicaments may be administered for oral administration, such as solid compositions, liquid compositions, and for parenteral administration, such as injections, liniments or suppositories, ophthalmic solution, respiratory tonic *etc.*

Solid compositions for oral administration include compressed tablets, pills, capsules, dispersible powders and granules. Capsules include hard capsules and soft capsules. Compressed tablets include sublingual tablets, buccals, troches, buccal patches, oral rapid disintegration tablet, *etc.*

In such solid forms, one or more of the active compound(s) may be admixed with excipients, such as lactose, mannitol, glucose, microcrystalline cellulose, starch; binders, such as hydroxypropyl cellulose, polyvinylpyrrolidone or magnesium metasilicate aluminate; disintegrates, such as cellulose calcium glycolate; lubricants, such as magnesium stearate; stabilizing agents, and solution adjuvants, such as glutamic acid or aspartic acid; and prepared according to methods well known in normal pharmaceutical practice. The solid forms may, if desired, be coated with coating agents, such as sugar, gelatin, hydroxypropyl cellulose or hydroxypropylmethyl cellulose phthalate; or be coated with two or more films. And further, coating may include containment within capsules of absorbable materials such as gelatin.

Liquid forms for oral administration include pharmaceutically acceptable solutions, suspensions and emulsions, syrups and elixirs. In such forms, one or more of the active compound(s) may be dissolved, suspended or emulsified into diluent(s) commonly used in the art, such as purified water, ethanol or a mixture thereof. Besides such liquid forms may also comprise some additives, such as wetting agents, suspending agents, emulsifying agents, sweetening agents, flavoring agents, aroma, preservative or buffering agent.

The dosage forms of the parenteral administration preparations for external use include ointments, gels, creams, fomentations, patches, liniments, atomized agents, inhalations, sprays, aerosols, eye drops, nasal drops and the like. Such a preparation contains one or two or more active substances and is prepared by a well known method or a commonly employed formulation.

Atomized agents, inhalations and sprays may contain, in addition to a diluent commonly employed, a stabilizer such as sodium hydrogen sulfite, a buffering agent for imparting isotonicity, for example, an isotonic agent such as sodium chloride, sodium citrate or citric acid. Methods for producing a spray are described in detail in, for example, U.S. Patent No. 2,868,691 and U.S. Patent No. 3,095,355.

The inhalations for parenteral administration include aerosols, powders for inhalation and liquids for inhalation. Such inhalations may be dissolved or suspended in water or another adequate medium for use.

The inhalations may be prepared in accordance with a well known method.

For example, liquid for inhalation may be, if necessary, prepared by appropriately selecting a preservative, such as benzalkonium chloride, paraben; a colorant, a buffering agent, such as sodium phosphate, sodium acetate; an isotonic agent, such as sodium chloride, concentrated glycerin; a thickener, such as carboxyvinyl polymer; an absorption promoter, and the like.

Powders for inhalation may be prepared, if necessary, by appropriately selecting a lubricant, such as stearic acid and its salt; a binder, such as starch, dextrin; an excipient, such as lactose, cellulose; a colorant, a preservative, such as benzalkonium chloride, paraben; an absorption promoter, and the like.

When the liquids for inhalation are administered, a sprayer (atomizer, nebulizer) is usually used. When the powders for inhalation are used, an inhalation administration apparatus for powder agents is usually used.

The injections for parenteral administration include solutions, suspensions, emulsions and solid injections to be dissolved or suspended before use. Such an injection is used by dissolving, suspending or emulsifying one or more active substances in a solvent. The solvent includes, for example, distilled water for injection, physiological saline, vegetable oils, alcohols such as propylene glycol, polyethylene glycol and ethanol, and mixtures thereof. The injection may further contain a stabilizer, a dissolution aid (glutamic acid, aspartic acid, Polysorbate 80 (registered trademark), etc.), a suspending agent, an emulsifier, a soothing agent, a buffer, a preservative, and the like. Such an injection may be produced by sterilizing at the final step or employing an aseptic process. Alternatively, it is also possible that an aseptic solid product such as a freeze-dried product is produced and sterilized or dissolved in aseptic distilled water for injection or another solvent before use.

Other compositions for parenteral administration include suppositories and pessaries for vaginal administration which contain one or more active substances, and are prepared in accordance with common formulations,

### Brief Description of Drawing

Figure 1 shows a mean linear intercept by an oral administration of 1 and 30 mg/kg of the compound (1) twice a day.
Figure 2 shows a mean linear intercept by an oral administration of 100 mg/kg of the compound (1) twice a day.

### Best Mode for Carrying out the Invention

The compound of formula (I) is very useful for a prevention and/or treatment of COPD.

The effect of the compound of the present invention on COPD was explained with following experiments, but not to limit the present invention.

### Experiment 1

Test compounds were as follows.

### Compound (1):

N-hydroxy-5-hydroxy-2(S)-methyl-4(S)-(4-phenoxybenzoyl) aminopentanamide administered at 1 or 30 mg/kg/dose, and

### Compound (2):

N-hydroxy-5-ethoxymethyloxy-2(S)-methyl-4(S)-(4-phenoxybenzoyl) aminopentanamide administered at 1 or 100 mg/kg/dose.

Wistar male rats were anesthetized with ketamine (100 mg/2 mL/kg of ketaral 50 for animal use), and then the intratracheal intubation was performed with nozzles for mist generation attached with 1 mL syringe. Porcine pancreatic elastase (PPE; 200 µL/lung) was instilled into tracheal duct. Test compound was administered orally prior to 1 hour of the induction, then it was administered twice daily with an interval of more than 6 hours.

Four weeks later of induction, animals were killed by blood phlebotomy of abdominal aorta under the ketamine (100 mg/2 mL/kg) anesthesia. Then trachea was exposed, thoracotomy was done, and trachea and lungs were removed along with heart. JMS cut down tube C3 attached to 50 ml syringe filled with 10% of neutral buffered formalin for tissue fixation was inserted into trachea. Lungs were fixed with 10% formalin under the pressure of 25 cm H₂O. After extension fixation for 20-26 hours, thyroid gland, heart, thymus and periphery adipose tissues were removed. Then the biggest one of all lung lobes was collected and processed for histological sections. The mean linear intercept (MLI) of alveoli in the section was measured.

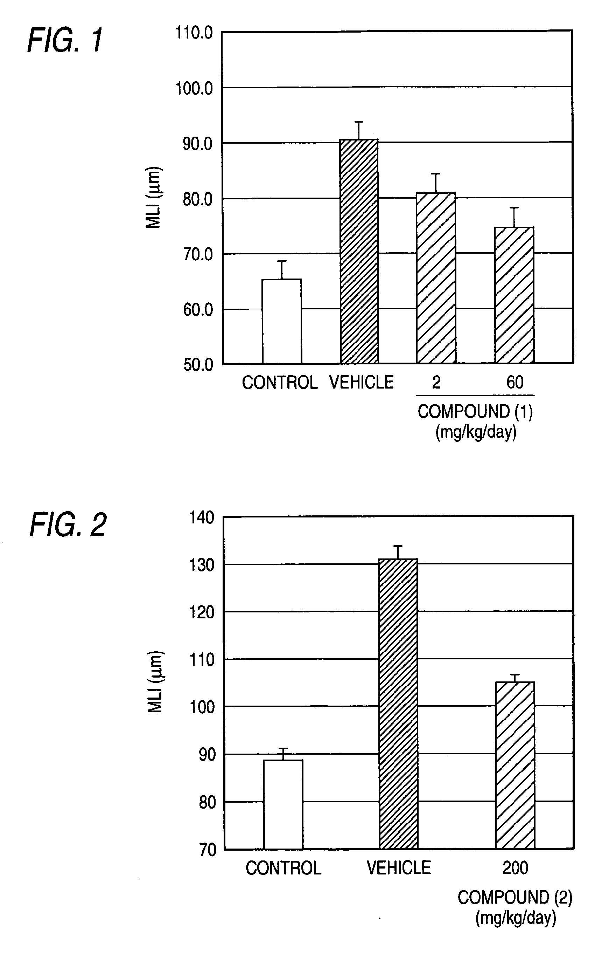

These results were shown in figure 1 and figure 2.

As is clear from figure 1 and figure 2, in the present model, test compounds significantly lowered MLI compared to vehicle group. Accordingly, it is determined that a compound of formula (I) is useful for COPD to suppress a progressive destruction of alveolar walls which was occurred by MMP.

### Formulation example 1

The following components were admixed in conventional method and punched out to obtain 10,000 tablets each containing 50 mg of active ingredient.
· N-hydroxy-5-hydroxy-2(S)-methyl-4(S)-(4-phenoxybenzoyl)aminopentanamide 500 g
· Carboxymethylcellulose calcium (disintegrating agent) 20 g
· Magnesium stearate (lubricating agent) 10 g
· Microcrystalline cellulose 470 g

### Formulation example 2

The following components were admixed in conventional method. The solution was sterilized in conventional manner, placed 5 ml portions into ampoules and freeze-dried to obtain 10,000 ampoules each containing 20 mg of the active ingredient.
· N-hydroxy-5-hydroxy-2(S)-methyl-4(S)-(4-phenoxybenzoyl)aminopentanamide 200 g
· Mannitol 2 kg
· Distilled water 50 L

### Industrial Applicability

The hydroxamic acid compound of formula (1), a salt thereof, a solvate thereof or a prodrug thereof is useful for a prevention and/or treatment of COPD.

## Claims

1. A prevention and/or treatment agent for chronic obstructive pulmonary disease comprising a hydroxamic acid compound of formula (I) 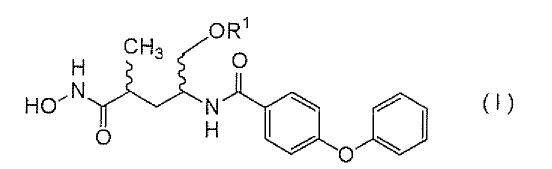 wherein R¹ is (i) hydrogen, (ii) C1-8 alkyl or (iii) C1-8 alkyl substituted by -OR², in which R² is (i) hydrogen, (ii) C1-8 alkyl, (iii) benzyl, or (iv) C1-8 alkyl substituted by C1-8 alkoxy;
a salt thereof, a solvate thereof or a prodrug thereof as active ingredient.

2. The prevention and/or treatment agent for chronic obstructive pulmonary disease according to claim 1, wherein the compound is N-hydroxy-5-hydroxy-2(S)-methyl-4(S)-(4-phenoxybenzoyl)aminopentanamide or N-hydroxy-5-ethoxymethyloxy-2(S)-methyl-4(S)-(4-phenoxybenzoyl)aminopentanamide.

3. The prevention and/or treatment agent for chronic obstructive pulmonary disease according to claim 2, which is administered in an amount of 12.5 mg to 600 mg per day.

4. A pharmaceutical composition which comprises a hydroxamic acid compound of formula (I) 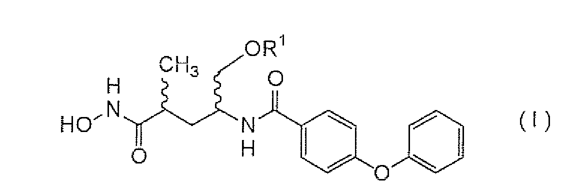 wherein all symbols are as claim 1; a salt thereof, a solvate thereof or a prodrug thereof, and one or at least two medicaments selected from anticholinergic agent, β₂ stimulator, theophylline products, leukotriene receptor antagonist, thromboxane synthetase inhibitor, thromboxane A₂ receptor antagonist, mediator release inhibitor, antihistamic agent, xanthine derivative, cytokine inhibitor, prostaglandins, forskolin, phosphodiesterase inhibitor, elastase inhibitor, aerosol steroid, expectorant, antibacterial agent.

5. Use of a hydroxamic acid compound of formula (I) 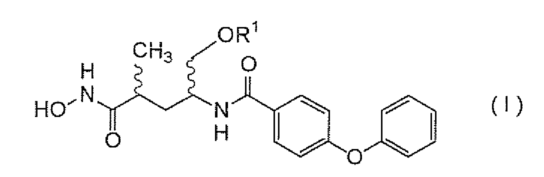 wherein all symbols are as claim 1, a salt thereof, a solvate thereof or a prodrug thereof for the manufacture of a medicament for prevention and/or treatment of chronic obstructive pulmonary disease.

6. The use according to claim 5, wherein the compound is N-hydroxy-5-hydroxy-2(S)-methyl-4(S)-(4-phenoxybenzoyl)aminopentanamide or N-hydroxy-5-ethoxymethyloxy-2(S)-methyl-4(S)-(4-phenoxybenzoyl)aminopentanamide.

7. A method for prevention and/or treatment of chronic obstructive pulmonary disease in a mammal, which comprises administering to a mammal an effective amount of a hydroxamic acid compound of formula (I) 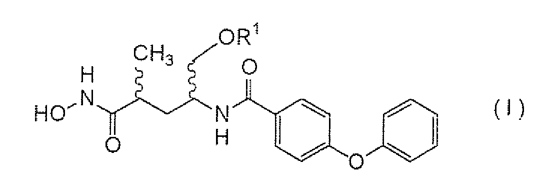 wherein all symbols are as claim 1; a salt thereof, a solvate thereof or a prodrug thereof.

8. Te method for prevention and/or treatment according to claim 7, wherein the compound is N-hydroxy-5-hydroxy-2(S)-methyl-4(S)-(4-phenoxybenzoyl)aminopentanamide or N-hydroxy-5-ethoxymethyloxy-2(S)-methyl-4(S)-(4-phenoxybenzoyl)aminopentanamide.
